# EUROPÄISCHE PATENTANMELDUNG

(11) **EP 3 388 962 A1**
(43) Veröffentlichungstag der Anmeldung: **17.10.2018**
(21) Anmeldenummer: 17166063.2
(22) Anmeldetag: 11.04.2017
(51) Int. Cl.: G06F 19/00, A61B 6/00

(54) **STEUERVERFAHREN EINES BILDGEBENDEN MEDIZINISCHEN SYSTEMS**

(71) Anmelder: Siemens Healthcare GmbH, 91052 Erlangen (DE)
(72) Erfinder: Kohle, Sven, 91056 Erlangen (DE); Kröll, Maria, 91052 Erlangen (DE); Prause, Andreas, 90451 Nürnberg (DE)

(57) **Zusammenfassung**

Die vorliegende Erfindung betrifft ein Steuerverfahren für eine Bildschirmanzeige eines bildgebenden medizinischen Systems, mit den Schritten eines Erfassens (S101) eines Bilddatensatzes eines Patienten; eines Vergleichens (S102) des erfassten Bilddatensatzes mit einer Anzahl vorgespeicherter Bilddatensätze, denen jeweils Layout-Parameter für die Bildschirmanzeige zugeordnet sind; und eines Anzeigens (S103) des erfassten Bilddatensatzes mit den Layout-Parametern jenes vorgespeicherten Bilddatensatzes, der die größte Ähnlichkeit zu dem erfassten Bilddatensatz aufweist.

## Beschreibung

Die vorliegende Erfindung betrifft ein Steuerverfahren für die Bildschirmanzeige eines bildgebenden medizinischen Systems und ein bildgebendes medizinisches System.

Die anwenderspezifische Anordnung und Strukturierung von Bilddaten auf einer Bildschirmanzeige, die an einem bildgebenden medizinischen System gewonnen werden, kann nur anhand umfangreicher Definitionen auf komplexer technischer Ebene durchgeführt werden.

Es ist die Aufgabe der vorliegenden Erfindung, Bilddaten automatisch für eine Bildschirmanzeige zu strukturieren, ohne dass umfangreiche Definitionen auf komplexer technischer Ebene durchgeführt werden müssen.

Diese Aufgabe wird durch Gegenstände nach den unabhängigen Ansprüchen gelöst. Vorteilhafte Ausführungsformen sind Gegenstand der abhängigen Ansprüche, der Beschreibung und der Figuren.

Gemäß einem ersten Aspekt wird die Aufgabe durch ein Steuerverfahren für eine Bildschirmanzeige eines bildgebenden medizinischen Systems gelöst, mit den Schritten eines Erfassens eines Bilddatensatzes eines Patienten; eines Vergleichens eines erfassten Bilddatensatzes mit einer Anzahl vorgespeicherter Bilddatensätze, zu denen jeweils Layout-Parameter für die Bildschirmanzeige zugeordnet sind; und eines Anzeigens des erfassten Bilddatensatzes mit den Layout-Parametern des vorgespeicherten Bilddatensatzes, der die größte Ähnlichkeit zu dem erfassten Bilddatensatz aufweist. Der Bilddatensatz wird beispielsweise mittels einer Pulssequenz in einem Magnettomographiegerät erzeugt oder mittels einer Auswertung einer Vielzahl, aus verschiedenen Richtungen aufgenommenen Röntgenaufnahmen, in einem Computertomographiegerät. Die Ähnlichkeit wird durch einen Vergleich des erfassten Bilddatensatzes mit jedem einzelnen der vorgespeicherten Bilddatensätze ermittelt. Die Ähnlichkeit lässt sich beispielsweise mittels eines Korrelationsparameters oder anderen Ähnlichkeitswertes bestimmen. Derjenige der vorgespeicherten Bilddatensätze, der den größten Korrelationsparameter oder Ähnlichkeitswert zu dem erfassten Bilddatensatz aufweist, weist auch die größte Ähnlichkeit zu diesem auf. Dadurch wird beispielsweise der technische Vorteil erreicht, dass sich eine schnelle, einfache und übersichtliche Strukturierung der Bilddatensätze erreichen lässt.

In einer technisch vorteilhaften Ausführungsform des Verfahrens wird die Ähnlichkeit zwischen dem erfassten Bilddatensatz und den vorgespeicherten Bilddatensätzen anhand von DICOM-Attributen bestimmt. Dadurch wird beispielsweise der technische Vorteil erreicht, dass sich eine Ähnlichkeitsanalyse mit geringem Rechenaufwand durchführen lässt.

In einer weiteren technisch vorteilhaften Ausführungsform des Verfahrens wird die Ähnlichkeit zwischen dem erfassten Bilddatensatz und den vorgespeicherten Bilddatensätzen auf Basis einer Bildanalyse durchgeführt. Dadurch wird beispielsweise der technische Vorteil erreicht, dass sich eine Ähnlichkeitsanalyse mit hoher Zuverlässigkeit durchführen lässt.

In einer weiteren technisch vorteilhaften Ausführungsform des Verfahrens sind die Layout-Parameter für den erfassten Bilddatensatz veränderbar. Dadurch wird beispielsweise der technische Vorteil erreicht, dass sich ein Layout der Bilddaten benutzerspezifisch verändern lässt.

In einer weiteren technisch vorteilhaften Ausführungsform des Verfahrens kann der erfasste Bilddatensatz zusammen mit den zugeordneten Layout-Parametern zu den vorgespeicherten Bilddatensätzen hinzugefügt werden. Dadurch werden die vorgespeicherten Bilddatensätze um einen Bilddatensatz erweitert. Dadurch wird beispielsweise der technische Vorteil erreicht, dass das veränderte Layout für die zukünftige Darstellung weitere Bilddatensätze verwendet werden kann.

In einer weiteren technisch vorteilhaften Ausführungsform des Verfahrens sind die Layout-Parameter durch ein Drag-and-Drop-Verfahren auf der Bildschirmanzeige veränderbar. Das Drag-and-Drop-Verfahren dient zur Bedienung grafischer Benutzeroberflächen von Computern durch das Bewegen grafischer Elemente mittels eines Zeigegerätes. Ein Element wie z. B. ein Piktogramm kann damit gezogen und über einem möglichen Ziel losgelassen werden. Dadurch wird beispielsweise der technische Vorteil erreicht, dass sich das Layout einfach und schnell anpassen lässt.
In einer weiteren technisch vorteilhaften Ausführungsform des Verfahrens steuern die Layout-Parameter die zeitliche Abfolge einer Anzeige der Bilddaten des Bilddatensatzes auf der Bildschirmanzeige. Dadurch wird beispielsweise der technische Vorteil erreicht, dass sich eine besonders geeignete Abfolge der Bilddaten erreichen lässt.

In einer weiteren technisch vorteilhaften Ausführungsform des Verfahrens werden die vorgespeicherten Bilddatensätze auf einem externen Datenspeicher bereitgestellt, auf den eine Mehrzahl von bildgebenden medizinischen Systemen Zugriff hat. Der externe Datenspeicher ist beispielsweise über ein Datennetz verbunden, wie eine Cloud zum Speichern von Daten im Internet. Dadurch wird beispielsweise der technische Vorteil erreicht, dass auf ein Vorwissen vieler Anwender zurückgegriffen werden kann, um ein geeignetes Layout zu finden.

In einer weiteren technisch vorteilhaften Ausführungsform des Verfahrens werden die Layout-Parameter automatisch auf Basis eines eingestellten Scan-Protokolls ausgewählt. Dadurch wird beispielsweise der technische Vorteil erreicht, dass sich das Layout auch ohne erfassten Bilddatensatz konfigurieren lässt.

In einer weiteren technisch vorteilhaften Ausführungsform des Verfahrens wird ein Scan-Protokoll einem Segment der Bildschirmanzeige zugeordnet. Dadurch wird beispielsweise der technische Vorteil erreicht, dass Scanprotokolle flexibel in benachbarten Segmenten der Bildschirmanzeige angeordnet werden können.

In einer weiteren technisch vorteilhaften Ausführungsform des Verfahrens ist das bildgebende medizinische System ein Magnetresonanztomografiegerät oder ein Computertomografiegerät. Dadurch wird beispielsweise der technische Vorteil erreicht, dass besonders geeignete bildgebende Geräte verwendet werden.

Gemäß einem zweiten Aspekt wird die Aufgabe durch ein bildgebendes medizinisches System gelöst, mit einer Erfassungsmodalität zum Erfassen eines Bilddatensatzes eines Patienten mittels einer Pulssequenz; einem Vergleichsmodul zum Vergleichen des erfassten Bilddatensatzes mit einer Anzahl vorgespeicherter Bilddatensätze, zu denen jeweils Layout-Parameter für die Bildschirmanzeige zugeordnet sind; und einem Steuermodul zum Anzeigen des erfassten Bilddatensatzes mit den Layout-Parametern des vorgespeicherten Bilddatensatzes, der die größte Ähnlichkeit zu dem erfassten Bilddatensatz aufweist. Durch das bildgebende medizinische System werden die gleichen Vorteile wie durch das Verfahren nach dem ersten Aspekt erreicht.

Gemäß einem dritten Aspekt wird die Aufgabe durch ein Computerprogramm mit Programmabschnitten zum Durchführen der Verfahrensschritte nach dem ersten Aspekt gelöst, wenn das Computerprogramm auf einem Computer laufen gelassen wird.

Gemäß einem vierten Aspekt wird die Aufgabe durch einen Datenspeicher mit einem Computerprogramm nach dem dritten Aspekt gelöst.

Ausführungsbeispiele der Erfindung sind in den Zeichnungen dargestellt und werden im Folgenden näher beschrieben.

Es zeigen:
- Fig. 1: ein Magnetresonanztomografiegerät;
- Fig. 2: eine Sicht auf eine Bildschirmanzeige;
- Fig. 3: eine Sicht auf eine Bildschirmanzeige;
- Fig. 4: ein Blockdiagramm eines Verfahrens; und
- Fig. 5: eine schematische Ansicht eines bildgebenden medizinischen Systems.

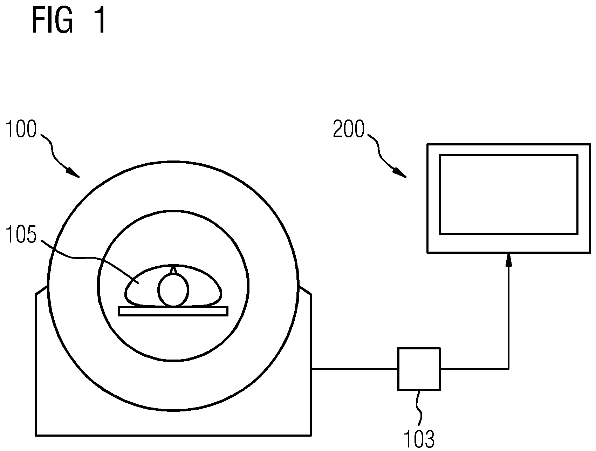

Fig. 1 zeigt ein Magnetresonanztomografiegerät 100 als bildgebendes medizinisches System. Das Magnetresonanztomografiegerät 100 dient zur bildgebenden medizinischen Untersuchung eines Patienten 105. Die Magnetresonanztomografie (MRT) verwendet ein externes Magnetfeld und die Wirkung, die eine Einstrahlung von Hochfrequenzimpulsen auf die Atomkerne des Körpergewebes des Patienten 105 besitzt. Die Atome werden mittels einer Pulssequenz in einen angeregten Zustand überführt. Beim Übergang der Atome vom angeregten in den Grundzustand (Relaxation) senden die Atome elektromagnetische Strahlung aus, die vom Magnetresonanztomografiegerät 100 als Resonanzsignal registriert werden kann.

Die Eigenschaften dieses Resonanzsignals hängen von der Pulssequenz und dem Körpergewebe ab, von dem das Resonanzsignal emittiert wird. Mit der Magnetresonanztomografie können Bilddatensätze 103 des Körpers gewonnen werden, die beispielsweise Schnittbilder umfassen und die eine Beurteilung krankhafter Veränderungen des Patienten 105 erlauben. Diese Bilddatensätze 103 lassen sich auf einer Bildschirmanzeige 200 anzeigen.

Benutzer des Magnetresonanztomografiegeräts 100, wie beispielsweise Radiologen, sind bestrebt, dass die erfassten Bilddatensätze 103 in einer vorgegebenen Reihenfolge und mit vorgegebenen Layout-Einstellungen auf der Bildschirmanzeige 200 angezeigt werden. Dabei sollten die Bilddatensätze 103 derart angeordnet und darstellt werden, dass die Diagnose schnell und zuverlässig vom Radiologen gestellt werden kann. Allerdings kann sich je nach Untersuchungsregion des Patienten und der radiologischen Modalität die zweckmäßige Darstellung und Anordnung der Bilddaten für diesen Zweck unterscheiden.

Zum Beispiel möchte ein Radiologe für eine standardmäßige Kopfuntersuchung, dass eine mit der Relaxationszeit T1 gewichtete Bildserie (T1-Bildserie) im linken oberen Bereich der Bildschirmanzeige 200 angezeigt wird und eine mit der Relaxationszeit T2 gewichtete Bildserie (T2-Bildserie) zum Vergleich daneben im rechten oberen Bereich der Bildschirmanzeige 200 angezeigt wird. Im Falle einer Onkologie-Untersuchung des Kopfes soll hingegen neben der T1-Bildserie eine weitere T1-Bildserie nach einer Kontrastmittelgabe erscheinen und die T2-Bildserie in der zweiten Zeile unter den T1-Bildserien erscheinen. In einem anderen Fall möchte der Radiologe für eine Computertomografieuntersuchung ein Volumen mit einem Weichteilfenster und ein Volumen mit einem Knochenfenster nebeneinander sehen. Gleiches gilt für die eine zeitlich-strukturierte, logische Abfolge der Bilddaten. Die Gesamtheit einer strukturierten Anzeige des Bilddatensatzes 103 wird Sicht genannt. Die Sicht betrifft sowohl die Anordnung der Bilddaten auf der Bildschirmanzeige 200 als auch die Abfolge der Bilddaten auf der Bildschirmanzeige 200.

Daher ist es vorteilhaft, die Bilddatensätze 103 automatisch für die Bildschirmanzeige 200 zu strukturieren, ohne dass umfangreiche Definitionen auf komplexer technischer Ebene durchgeführt werden müssen.

Zu diesem Zweck erzeugt der Radiologe nach einer Untersuchung bei einem ersten Lesen eines Falles interaktiv eine strukturierte Sicht nach persönlichen Vorstellungen anhand eines bereits vorhandenen Bilddatensatzes 103. Hierbei werden Layout-Parameter festgelegt. Die Layout-Parameter geben an, auf welche Weise der Bilddatensatz auf einer Anzeige angezeigt wird. Die Layout-Parameter umfassen beispielsweise die Anzahl der Bildsegmente auf dem Bildschirm (2x2 oder 3x3), eine Zuordnung von bestimmten Bildserien zu den Segmenten, eine Reihenfolge mehrerer Layouts, eine Darstellung der Bilddaten innerhalb einer Bildserie (Knochenfenster, Weichteilfenster), Zoom-Faktoren, Synchronisationseinstellungen oder bestimmte Blickwinkel für ein dreidimensionales Volumen. Gleiches gilt für mehrere Studien beim Vergleich mit Voruntersuchungen. Im Allgemeinen können die Layout-Parameter alle Parameter umfassen, mit denen eine bestimmte Anzeige des Bilddatensatzes 103 gesteuert wird.

Die Zuordnung der jeweiligen Bilddaten zu Bildsegmenten der Bildschirmanzeige 200 basiert dabei auf DICOM-Attributen (DICOM - Digital Imaging and Communications in Medicine). Die DICOM-Attribute sind im DICOM-Standard festgelegt, der ein offener Standard zur Speicherung und zum Austausch von Informationen im medizinischen Bilddatenmanagement ist. Das DICOM-Attribut wird über eine festgelegte achtstellige Hexadezimalzahl, ein sogenanntes Data-Tag definiert.

Die ersten vier Stellen des Data-Tags definieren die Zugehörigkeit des Attributes zu einer bestimmten Gruppe (wie beispielsweise File Meta Information), die weiteren vier Stellen bestimmen das Element. Zur besseren Lesbarkeit wird ein DICOM Data Tag normalerweise in der Form (xxxx, yyyy) mit einem Komma in der Mitte dargestellt.

In einem weiteren Schritt kann zusätzlich ein selbstlernendes System eine Bildanalyse durchführen und korrigierend eingreifen, falls ein DICOM-Wert mit einem bestimmten Bildwert nicht übereinstimmt. Beispielsweise steht in einem Namen der aufgenommenen Pulssequenz noch "T1", obwohl es sich aufgrund der geänderten Bildparameter um ein T2-Kontrastbild handelt. Diese Eigenschaft kann teilweise auch aus weiteren DICOM-Tags zusätzlich zum Sequenznamen abgelesen werden. Für den Radiologen ist letztlich aber der Bildeindruck entscheidend.

Das Magnetresonanztomografiegerät 100 ist als ein selbstlernendes System (Deep Learning) konfiguriert und lernt schrittweise die entscheidenden Layout-Parameter mit. Nach einer initialen, manuellen und interaktiven Strukturierung des Bilddatensatzes 103 kann der Radiologe einen Speicher-Knopf betätigen, so dass der Bilddatensatz 103 dieser Untersuchung (Studie) mit den gewählten Layout-Parametern verknüpft ist. Wenn der Radiologe eine neue Studie lädt, versucht das Magnetresonanztomografiegerät 100 anhand von Identitäts- und Ähnlichkeitsregeln auf Basis des Bilddatensatzes 103 herauszufinden, ob es bereits eine vordefinierte Sicht für diese Untersuchung gibt.

Wird keine vordefinierte Sicht gefunden, lädt das Magnetresonanztomografiegerät 100 eine Standardsicht. Der Radiologe kann manuell eine der vordefinierten Sichten ebenfalls dem Bilddatensatz 103 dieser Studie zuordnen und kleine Anpassungen vornehmen. Das Magnetresonanztomografiegerät 100 speichert diese Anpassungen als mögliche Varianten ab und lernt zusätzlich, auch die neue Studie als ähnliche Studie zu identifizieren.

Mit jeder neuen Studie, die der Radiologe befundet, lernt das Magnetresonanztomografiegerät 100, welche Layout-Parameter einer Studie für die Zuordnung bestimmter Sichten relevant sind und welche Varianten innerhalb einer Sicht auf die einzelnen Daten innerhalb einer Studie anzuwenden sind. Wird eine Studie als ähnlich zu einer anderen Studie erkannt, die bereits mit einer vordefinierten Sicht versehenen ist, wird die vordefinierte Sicht geladen und mit den vorhandenen Bilddaten befüllt.

Der Radiologe kann Bilddaten, die nicht automatisch zugeordnet werden können, manuell in einzelnen Segmenten der Bildschirmanzeige 200 anordnen und so Varianten der Sicht im Magnetresonanztomografiegerät 100 anlegen, die alle als zugehörig gespeichert zu einer bestimmten Studienart sind. Die Bilddatensätze 103 werden nach dem Ähnlichkeitsprinzip in vorhandene Segmente der Bildschirmanzeige 200 geladen und mit den entsprechenden Attributen versehen. Je mehr Studien befundet worden sind, desto robuster und zuverlässiger ist eine Zuordnung zu Sichten und deren Detailgestaltung.

Der Radiologe kann dem Magnetresonanztomografiegerät 100 durch explizites Speichern mitteilen, welche Einstellungen gespeichert werden sollen. In diesem Fall zeigt das Magnetresonanztomografiegerät 100 an, ob eine neue Sicht erstellt worden ist oder eine Variante einer bestehenden Sicht erzeugt worden ist. Der Radiologe kann diese automatisierte Zuordnung ändern und die Namen der Sichten frei vergeben.

In einer administrativen Ansicht können alle Attribute, wie beispielsweise Fenster-Werte, Zoom-Faktoren oder Bildfilter, im Detail aufgelistet werden und entsprechend an- oder abgewählt werden. In einer weiteren Ausbaustufe für mehrere Benutzer einer Abteilung kann eine Variante für Administratoren und eine weitere Variante für reguläre Benutzer existieren. Beispielsweise können lediglich Einstellungen von einem Administrator berücksichtigt und abgespeichert werden. Reguläre Benutzer haben jedoch keinen Einfluss auf den Regelaufbau des selbstlernenden Systems. Im Allgemeinen können die Einstellungen je System, je Nutzer oder für alle Nutzer generell abgespeichert werden und das System lernt verschiedene Untersuchungsarten zu unterscheiden.

Das manuelle Setzten von Layout-Parametern, die die Visualisierung von medizinischen Bilddaten bestimmen, wird durch das selbstlernende System automatisiert. Vordefinierte Sichten und automatisierte Zuordnungen von Sichten zu radiologischen Studien können die Leseeffizienz und den diagnostischen Standard erhöhen. Im Sinne der Qualität profitieren besonders radiologische Anfänger vom Vorwissen der erfahrenen Radiologen, wie man eine bestimmte Studie am besten befunden sollte. Die Performance in der Datenanalyse, Zuordnung und die Geschwindigkeit, in dem das System die Zuordnungen lernen kann, wird durch die einfache Ebene der DICOM- oder Header-Parameter erhöht. Erst für eine feine Optimierung der Zuordnung wird auf eine Bildanalyse zurückgegriffen. Die Anzeige der erfolgten Zuordnungen erlaubt es dem Nutzer bei Bedarf in einer Steuerung zu bleiben und das System nach seinen Bedürfnissen auch explizit auszusteuern.

In Magnetresonanztomografiegeräten 100 existiert oftmals eine Datenbasis an strukturierten Lese-Protokollen, d.h. Studien mit zugeordneten Arbeitsabläufen (Workflows) und darin enthaltenen Layouts mit Zuordnungen von Bilddaten zu bestimmten Segmenten. Diese Lese-Protokolle können einem selbstlernenden System (Deep Learning) mit dazu jeweils passendem Bildmaterial an radiologischen Untersuchungen zugeführt werden.

Dadurch kann das Magnetresonanztomografiegerät 100 erkennen, welche DICOM-Parameter oder sonstigen Header-Parameter der Bilddatensätze 103 für die Zuordnung von Serien zu bestimmten Segmenten und für die Zuordnung von Studien zu einem bestimmten Lese-Protokoll relevant sind. Das Magnetresonanztomografiegerät 100 kann weitere Muster ableiten, welche Studien jeweils ein eigenes Leseprotokoll haben oder welche Hauptvarianten pro Studie es gibt. Es kann Muster über die Charakteristika der einzelnen Leseprotokolle je Studie ableiten.

Zusätzlich werden die tatsächlichen radiologischen Bilddaten dem System übergeben, das folglich Bilddaten und Header-Daten kennt und entsprechend zuordnen kann. Bei unklaren Header-Daten kann das System auf eine zusätzliche Bildanalyse zurückgreifen und damit die Treffsicherheit einer korrekten Zuordnung von Bilddaten innerhalb eines Leseprotokolls erhöhen.

Dabei sollen bestimmte Bilddaten in bestimmte Segmente geordnet werden, bestimmte Layouts in bestimmte Reihenfolgen gebracht werden und Bilddaten innerhalb der einzelnen Segmente visualisiert werden. Die Visualisierung kann über ein Lernen der Regeln zum sichtbaren Bildeindruck zuverlässiger erfolgen als über die reine Analyse von Header-Daten.

Wenn ein solches System von einem Radiologen das erste Mal verwendet wird, kann eine aufgenommene Studie basierend auf Regeln, die über ein Vorab-Training erarbeiteten worden sind, in ein vordefiniertes Leseprotokoll geladen werden, das die Erwartungen des Radiologen bereits erfüllt.

Das System erkennt beispielsweise einen Bildeindruck eines Magnetresonanzbildes bei einer Verwendung von Kontrastmittel und ordnet ein Magnetresonanzbild ohne Verwendung von Kontrastmittel zum Vergleich daneben an. Beispielsweise ordnet das System ein CT-Bild aus einem CT-Lungenscan zusammen mit einem CT-Lungenfenster an oder das System fenstert ein Magnetresonanzbild eines Herzens derart, dass eine bestimmte Herzstruktur maximal im Bild dargestellt ist.

Zusätzlich kann das System durch eine modalitäts-spezifische Datenbank an Bildeigenschaften trainiert werden. Zum Beispiel gibt es eine Datenbank an MR-Sequenzen mit dazu abgelegtem Bildmaterial. Zusammen mit einer Auswahl an vordefinierten, besonders relevanten DICOM-Parametern, kann dies einen initialen Input für das selbstlernende System darstellen, um initial zu erlernen, welche Parameter zu welchem Bildeindruck führen.

Dadurch kann die vorhandene Datenbasis an radiologischen Leseabläufen einem selbstlernenden System zugeführt werden, um eine manuelle Konfiguration durch den Benutzer zu vermeiden. Ein strukturiertes Lesen erhöht die Effizienz in den Befunden, verringert diagnostische Fehler und unterstützt eine Standardisierung.

Ein selbstlernendes System, das eine vorhandene Datenbasis verwendet, ist effizient, da der Benutzer bei der ersten Verwendung des Systems auf ein Vorwissen aus der radiologischen Praxis aufbauen kann. Dadurch wird die Effizienz erhöht und das selbstlernende System wir lediglich an die institutionsspezifischen und Nutzer-spezifischen Besonderheiten angepasst.

Radiologen können sich eine Anzeige der Bilddaten zum Befunden oder Lesen vordefiniert einrichten. Beispielsweise soll für einen Radiologen eine Kopfuntersuchung mit einer bestimmten Fragestellung immer in der gleichen Art und Weise zum Lesen aufbereitet werden, d.h. den gleichen Layouts mit den gleichen Bilddaten an den gleichen Stellen und der gleichen Reihenfolge an Layouts, die aufeinander folgen.

Die Festlegung, welche Bilddaten in welchen Segmenten der Bildschirmanzeige 200 angezeigt werden sollen, kann entweder direkt durch das Ziehen der tatsächlichen Bildserien auf das Zielsegment oder über eine komplexe Konfiguration eines regulären Ausdrucks direkt in dem Zielsegment erreicht werden.

In einem neu eingerichteten System 100 sind jedoch die erforderlichen Bilddaten oftmals noch nicht verfügbar. Allerdings sind an dem Magnetresonanztomografiegerät 100 bereits Scan-Protokolle verfügbar, die für die gewünschte Untersuchung verwendet werden. Ein Scan-Protokoll umfasst die Scanparameter für die Akquisition von CT- und MR-Daten, wie beispielsweise Pulsfolgen oder Aufnahmewinkel.

Die Zuordnung von Bilddaten zu Segmenten innerhalb des Layouts ist daher nicht nur anhand der Bilddaten als solchen möglich, sondern ebenfalls anhand der ausgewählten Scan-Sequenzen oder Scan-Protokolle.

Dazu werden im Leseumfeld diejenigen Scan-Programme bereitgestellt, die am jeweiligen Magnetresonanztomografiegerät 100 verfügbar sind. In einer Simulation können die Scan-Sequenzen in die resultierenden Bildserien oder darauf basierende Datenrollen übersetzt werden.

Der Radiologe kann nun an Stelle der tatsächlichen Bilder die Scan-Sequenzen des Magnetresonanztomografiegerätes 100 auswählen und auf das gewünschte Segment der Bildschirmanzeige 200 ziehen. Sind mehrere Magnetresonanztomografiegeräte 100 mit unterschiedlichen Scan-Sequenzen verfügbar, werden mehrere Ergebnisserien auf ein Segment gezogen. Darüber ergeben sich mögliche Alternativen für ein Segment. Die Reihenfolge, in der die Namen abgelegt wurden, bestimmt gleichzeitig die Reihenfolge für eine mögliche Anzeige und damit die Priorität.

Wenn noch keine Bilddaten erfasst worden sind, kann das Einrichten eines Leseprotokolls daher anhand von Scan-Protokollen erfolgen. Das Leseprotokoll kann von Administratoren unabhängig von den verfügbaren Bilddatensätzen und auf Basis der vorhandenen Scan-Protokolle bereits vorbereitet eingerichtet werden.

Abweichungen zwischen verschiedenen Magnetresonanztomografiegeräten können bereits vorab berücksichtigt werden, so dass diese nicht nach und nach in der Konfiguration nachgezogen werden müssen, wenn entsprechende Bilddaten verfügbar sind.

Das Verfahren kann durch ein Computerprogramm mit Programmabschnitten zum Durchführen der Verfahrensschritte implementiert sein, wenn das Computerprogramm auf einem Computer laufen gelassen wird. Der Computer umfasst einen Datenspeicher zum Speichern des Computerprogramms und einen Prozessor zum Verarbeiten des Computerprogramms. Das Computerprogramm kann wiederrum auf einem externen Datenspeicher gespeichert sein, von das Computerprogramm in den internen Datenspeicher des Computers geladen werden kann. Der externe Datenspeicher ist beispielsweise eine CD-ROM, ein USB-Flash Speicher oder ein Speicherlaufwerk im Internet.

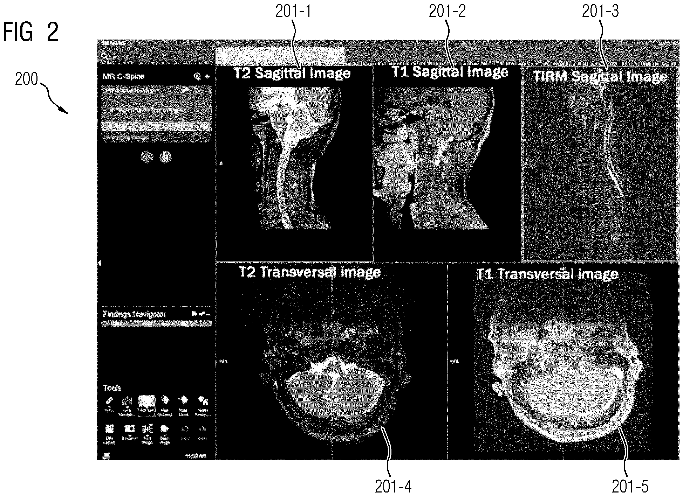

Fig. 2 zeigt eine Sicht auf die Bildschirmanzeige 200. Die Bildschirmanzeige 200 ist beispielsweise in unterschiedliche Segmente unterteilt. Die Segmente sind durch einzelne Teilbereiche der Bildschirmanzeige 200 gebildet.

In den Segmenten 201-1, ..., 201-5 als Teilbereichen der Bildschirmanzeige 200 werden jeweils unterschiedliche Bilddaten des Bilddatensatzes 103 dargestellt. Im oberen Bereich der Bildschirmanzeige 200 sind die Segmente 201-1, 201-2 und 201-3 nebeneinander angeordnet. Im unteren Bereich der Bildschirmanzeige 200 sind die Segmente 201-4 und 201-5 nebeneinander angeordnet. Die Anordnung der Segmente und der angezeigten Bilddaten erfolgt auf Basis zuvor ermittelter Layout-Parameter.

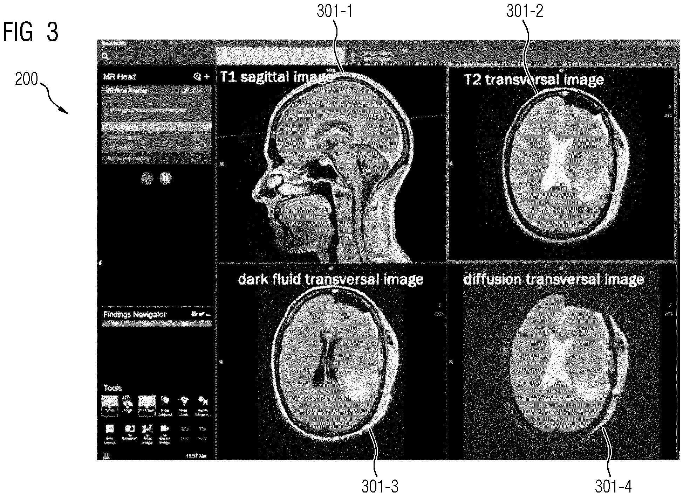

Fig. 3 zeigt eine weitere Sicht auf die Bildschirmanzeige 200. In den Segmenten 301-1, ..., 301-4 werden jeweils unterschiedliche Bilddaten des Bilddatensatzes 103 dargestellt. Im oberen Bereich der Bildschirmanzeige 200 sind die Segmente 301-1 und 301-2 nebeneinander angeordnet. Im unteren Bereich der Bildschirmanzeige 200 sind die Segmente 301-3 und 301-4 nebeneinander angeordnet. Die Anordnung der Segmente und der angezeigten Bilddaten erfolgt auf Basis zuvor ermittelter Layout-Parameter. Diese Layout-Parameter weisen andere Werte auf als die Layout-Paramater für die Sicht aus Fig. 2.

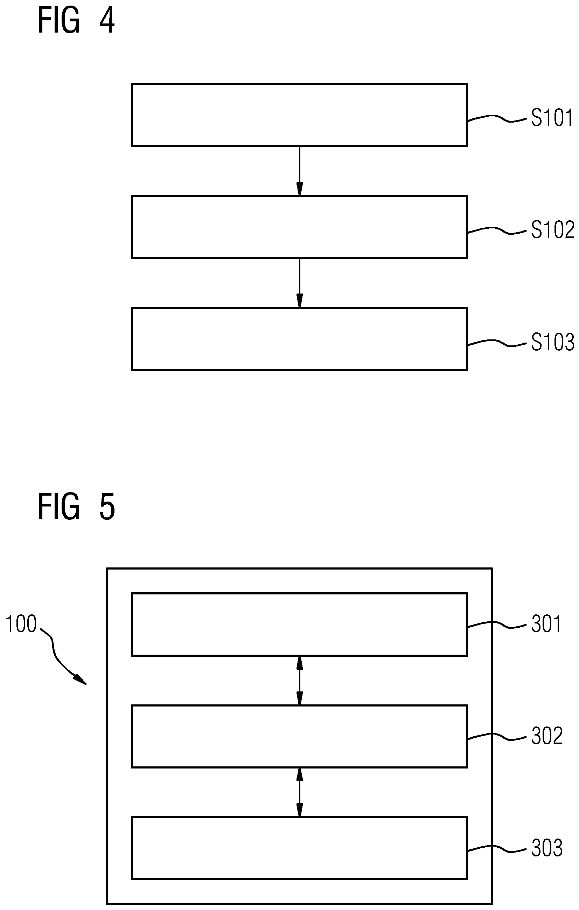

Fig. 4 zeigt ein Blockdiagramm Steuerverfahrens für die Bildschirmanzeige 200 des bildgebenden medizinischen Systems 100. In Schritt S101 wird zunächst der Bilddatensatz 103 eines Patienten mittels einer Pulssequenz erfasst. Der Bilddatensatz 103 umfasst unterschiedliche Bilddaten, wie beispielsweise Schnittbilder und DICOM- oder Header-Attribute.

In Schritt S102 wird der erfasste Bilddatensatzes 103 mit einer Anzahl vorgespeicherter Bilddatensätze verglichen, zu denen jeweils Layout-Parameter für die Bildschirmanzeige 200 zugeordnet sind. Der Vergleich umfasst beispielsweise eine Ähnlichkeitsanalyse, eine Korrelationsanalyse oder eine grafische Bildanalyse.

In Schritt S103 wird der erfasste Bilddatensatzes 103 mit den Layout-Parametern desjenigen vorgespeicherten Bilddatensatzes angezeigt, der die größte Ähnlichkeit zu dem erfassten Bilddatensatz 103 aufweist.

Fig. 5 zeigt eine schematische Ansicht eines bildgebenden medizinischen Systems. Das bildgebende medizinische System 100 umfasst eine Erfassungsmodalität 301 zum Erfassen eines Bilddatensatzes 103 eines Patienten mittels einer Pulssequenz.

Die Erfassungsmodalität 301 ist beispielsweise durch ein Magnetresonanztomografiegerät gebildet.

Ein Vergleichsmodul 302 dient zum Vergleichen des erfassten Bilddatensatzes 103 mit einer Anzahl vorgespeicherter Bilddatensätze, zu denen jeweils Layout-Parameter für die Bildschirmanzeige 200 zugeordnet sind. Das Vergleichsmodul 302 ist beispielsweise durch eine elektrische digitale Schaltung gebildet, die einen Datenspeicher zum Speichern der Bilddatensätze und einen Prozessor zum Verarbeiten der Bilddatensätze umfasst. Zusätzlich ist der Prozessor in der Lage, eine Ähnlichkeitsanalyse, eine Korrelationsanalyse oder eine grafische Bildanalyse durchzuführen.

Ein Steuermodul 303 dient zum Anzeigen des erfassten Bilddatensatzes 103 mit den Layout-Parametern des vorgespeicherten Bilddatensatzes, der die größte Ähnlichkeit zu dem erfassten Bilddatensatz 103 aufweist. Zu diesem Zweck umfasst das Steuermodul 303 ebenfalls eine elektrische digitale Schaltung, die die Bildschirmanzeige 200 auf Basis der Layout-Parameter derart steuert, dass der Bilddatensatz 103 in dem vorgesehen Layout angezeigt wird.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die Ansprüche gegeben und wird durch die in der Beschreibung erläuterten oder den Figuren gezeigten Merkmale nicht beschränkt.

## Patentansprüche

1. Steuerverfahren für eine Bildschirmanzeige (200) eines bildgebenden medizinischen Systems (100), mit:
- Erfassen (S101) eines Bilddatensatzes (103) eines Patienten (105);
- Vergleichen (S102) des erfassten Bilddatensatzes (103) mit einer Anzahl vorgespeicherter Bilddatensätze, zu denen jeweils Layout-Parameter für die Bildschirmanzeige (200) zugeordnet sind; und
- Anzeigen (S103) des erfassten Bilddatensatzes (103) mit den Layout-Parametern des vorgespeicherten Bilddatensatzes, der die größte Ähnlichkeit zu dem erfassten Bilddatensatz (103) aufweist.

2. Steuerverfahren nach Anspruch 1, wobei die Ähnlichkeit zwischen dem erfassten Bilddatensatz (103) und den vorgespeicherten Bilddatensätzen anhand von DICOM-Attributen bestimmt wird.

3. Steuerverfahren nach einem der vorangehenden Ansprüche, wobei die Ähnlichkeit zwischen dem erfassten Bilddatensatz (103) und den vorgespeicherten Bilddatensätzen auf Basis einer Bildanalyse durchgeführt wird.

4. Steuerverfahren nach einem der vorangehenden Ansprüche, wobei die Layout-Parameter für den erfassten Bilddatensatz (103) veränderbar sind.

5. Steuerverfahren nach Anspruch 4, wobei der erfasste Bilddatensatz (103) zusammen mit den zugeordneten Layout-Parametern zu den vorgespeicherten Bilddatensätzen hinzugefügt werden kann.

6. Steuerverfahren nach Anspruch 4 oder 5, wobei sich die Layout-Parameter durch ein Drag-and-Drop-Verfahren auf der Bildschirmanzeige (200) veränderbar sind.

7. Steuerverfahren nach einem der vorangehenden Ansprüche, wobei die Layout-Parameter die zeitliche Abfolge einer Anzeige der Bilddaten des Bilddatensatzes (103) auf der Bildschirmanzeige (200) steuern.

8. Steuerverfahren nach einem der vorangehenden Ansprüche, wobei die vorgespeicherten Bilddatensätze auf einem externen Datenspeicher bereitgestellt werden, auf den eine Mehrzahl von bildgebenden medizinischen Systemen (100) Zugriff hat.

9. Steuerverfahren nach einem der vorangehenden Ansprüche, wobei die Layout-Parameter automatisch auf Basis eines eingestellten Scan-Protokolls ausgewählt werden.

10. Steuerverfahren nach Anspruch 9, wobei ein Scan-Protokoll einem Segment (201-1, ..., 201-5) der Bildschirmanzeige (200) zugeordnet wird.

11. Steuerverfahren nach einem der vorangehenden Ansprüche, wobei das bildgebende medizinische System (100) ein Magnetresonanztomografiegerät oder ein Computertomografiegerät ist.

12. Bildgebendes medizinisches System (100), mit:
einer Erfassungsmodalität (301) zum Erfassen eines Bilddatensatzes (103) eines Patienten (105);
einem Vergleichsmodul (302) zum Vergleichen des erfassten Bilddatensatzes (103) mit einer Anzahl vorgespeicherter Bilddatensätze, zu denen jeweils Layout-Parameter für die Bildschirmanzeige (200) zugeordnet sind; und
einem Steuermodul (303) zum Anzeigen des erfassten Bilddatensatzes (103) mit den Layout-Parametern des vorgespeicherten Bilddatensatzes, der die größte Ähnlichkeit zu dem erfassten Bilddatensatz (103) aufweist.

13. Computerprogramm mit Programmabschnitten zum Durchführen der Verfahrensschritte nach einem der Ansprüche 1 bis 11, wenn das Computerprogramm auf einem Computer laufen gelassen wird.

14. Datenspeicher mit einem Computerprogramm nach Anspruch 13.
